# EUROPÄISCHE PATENTANMELDUNG

(11) **EP 1 072 581 A2**
(43) Veröffentlichungstag der Anmeldung: **31.01.2001**
(21) Anmeldenummer: 00115053.1
(22) Anmeldetag: 26.07.2000
(51) Int. Cl.: C07C 51/41, C07C 51/43, C07C 53/122, F26B 3/12

(54) **Verfahren zur Herstellung von Calciumpropionat und Vorrichtung zur Durchführung des Verfahrens**

(30) Priorität: 27.07.1999 DE 19935298
(71) Anmelder: BASF Aktiengesellschaft, 67063 Ludwigshafen (DE)
(72) Erfinder: Lockemann, Christian, Dr., 68163 Mannheim (DE); Lämmermann, Fritz, 67227 Frankenthal (DE); Margin, Albert, 67061 Ludwigshafen (DE)
(74) Vertreter: Isenbruck, Günter, Dr.

(57) **Zusammenfassung**

Gegenstand der Erfindung sind ein Verfahren zur Herstellung von pulverförmigem Calciumpropionat unter Verwendung eines konvektiven Trockners, bei dem eine feinteilige, pumpbare Suspension von Calciumpropionat in Wasser mit einem Calciumpropionat-Gehalt von größer oder gleich 30 % (w/w) einem konvektiven Trockner zugeführt wird und eine Vorrichtung zur Durchführung des Verfahrens mit einem Reaktor (1), einer Schlaufe (4), einer Dispergiermaschine (3) und gegebenenfalls einem Vorlagebehälter (8) mit einem Trockner (10).

## Beschreibung

Die Erfindung bezieht sich auf ein Verfahren zur Herstellung von Calciumpropionat (Ca(CH₃CH₂COO)₂) sowie auf eine Vorrichtung, die zur Durchführung des erfindungsgemäßen Verfahrens besonders geeignet ist.

Calciumpropionat Mᵣ=186,18 wird beispielsweise als Konservierungsstoff für Tierfutter verwendet oder als Arzneimittel für Haustiere. Calciumpropionat hat eine ausgeprägte fungiostatische Wirkung. Deshalb wird es auch Backwaren zur Schimmelvermeidung zugesetzt.

Das am häufigsten zur Herstellung von Calciumpropionat eingesetzte Verfahren umfaßt die Neutralisation von Propionsäure mit Calciumhydroxid in Wasser und eine anschließende Sprühtrocknung der so gebildeten wäßrigen Lösungen (Ullmann's Encyclopedia, 5. Auflage, Band A22, Seite 232, 1993, VCH-Verlag mbH Weinheim).

Bei der Durchführung des bekannten Verfahrens wird die Löslichkeitsgrenze für Calciumpropionat in Wasser, die unter typischen Bedingungen nicht ganz 30% (w/w) beträgt, nicht überschritten, da unter den Bedingungen des bekannten Verfahrens grobkristallines Calciumpropionat ausfällt. Das bekannte Verfahren arbeitet also stets mit wässrigen Lösungen.

Die Löslichkeit von Calciumpropionat ist Nachschlagewerken zu entnehmen (siehe R.C. Weast et al. 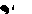 CRC Handbook of Chemistry and Physics", CRC Press, Boca Raton, Fl, 70. Auflage (1989), B-81; W.F. Linke, 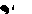 Solubilities of Inorganic and Metal-Organic Compounds", Van Nostrand Co., Princeton, NJ (1958), Vol. 1, Seite 511) und kann z.B. durch Streulichtmessung bestimmt werden.

Problematisch ist bei diesem bekannten Verfahren, daß aufgrund der geringen Löslichkeit von Calciumpropionat, bezogen auf die Calciumpropionatmenge, große Mengen Wasser verdampft werden müssen, was entsprechend umweltbelastend und kostenintensiv ist.

Eine weitere Möglichkeit zur Herstellung von Calciumpropionat besteht in der Kristallisation aus wäßriger Phase. Die Löslichkeit von Calciumpropionat ist nur schwach tempertaturabhängig und weist ein flaches Minimum bei 50°C auf. Kühlungskristallisation ist somit ungeeignet. Diskontinuierliche Verdampfungs-kristallisation ist zwar möglich, aber in bezug auf den Energieaufwand nicht vorteilhafter als Sprühtrocknung aus wäßrigen Lösungen.

Das Salz kann auch durch eine kontinuierliche Zugabe der Reaktanden (z.B. Propionsäure und Calciumhydroxid) und durch kontinuierlichen Abzug der ausgefallenen Kristalle erhalten werden (Verfahren nach Fa. Fleischmann's, Greenville, Texas, USA). Zwar weist dieses Verfahren nicht die geschilderten energetischen Nachteile auf, allerdings ist die Durchführung des Verfahrens problematisch, da eine Rückvermischung von Edukt (z.B. Calciumhydroxid) mit Calciumpropionat in Hinblick auf die Produktqualität vermieden werden muß. Dies setzt aufwendige Maßnahmen zur Kontrolle der Strömung im Kristallisator voraus.

Die bei der Kristallisation gebildeten Partikel werden in einer Zentrifuge oder mit einem Filter von der Lauge abgetrennt und anschließend getrocknet. Die abgetrennten, aber noch feuchten Kristalle zeigen eine starke Verbackungsneigung, was eine komplizierte Feststoff-Handhabung erforderlich macht. Es entstehen somit große und kompakte Kristalle, die eine schlechtere konservierende Wirkung zeigen als die durch Sprühtrocknung erhaltenen. Deswegen wird in der Regel ein nachgeschalteter aufwendiger Mahlschritt erforderlich sein.

Calciumpropionat kann auch durch lösungsmittelfreie Verfahren hergestellt werden.

In einer Verfahrensvariante wird Calciumhydroxid kontinuierlich mit Propion-säure in einem rotierenden, mit Keramikkugeln gefüllten Reaktionsrohr bei 140°C umgesetzt (Verfahren der BASF AG, Ludwigshafen). Die Bildung des Calciumpropionats erfolgt lösungsmittelfrei am Kalk, der lediglich an seiner Oberfläche vom Reaktanden Propionsäure angelöst wird und dabei reagiert. Das gebildete Reaktionswasser verdampft sofort und wird abgezogen. Nachteilig bei diesem Verfahren ist eine gewisse Störanfälligkeit des Reaktors, die Propionsäureverluste durch Verdampfung infolge lokaler Überhitzung und die starke Verklumpung des Produktes im Reaktor. Außerdem gelangt durch die mechanische Beanspruchung der Keramikkugeln Abrieb in den Reaktor und somit in das Produkt.

Weitere Varianten beruhen auf der lösungsmittelfreien Calciumpropionat-herstellung im Rührbett oder in der Wirbelschicht. Die Verfahren weisen ähnliche Nachteile auf.

Es ist somit Aufgabe der vorliegenden Erfindung, ein Verfahren zur Herstellung von Calciumpropionat anzugeben, das energetisch günstiger durchführbar ist als die bereits bekannten Verfahren und das keinen zusätzlichen Mahlschritt erfordert.

Die Lösung der Aufgabe besteht erfindungsgemäß in einem Verfahren zur Herstellung von pulverförmigem Calciumpropionat unter Verwendung eines konvektiven Trockners, bei dem eine feinteilige, pumpbare Suspension von Calciumpropionat in Wasser mit einem Calciumpropionat-Gehalt von größer oder gleich 30 % (w/w) einem konvektiven Trockner zugeführt wird.

Im Rahmen der Erfindung werden Prozentangaben in Gewichtsprozent (Massenbruch) angegeben.

Dem Verfahren liegt die Erkenntnis zugrunde, daß Calciumpropionat auch als stabile, wäßrige und pumpbare Kristallmaische (Calciumpropionat-Suspension) mit einem Calciumpropionatgehalt oberhalb der Löslichkeitsgrenze hergestellt und gefördert werden kann, ohne daß es zur Bildung grober Kristalle bei der Überschreitung der Löslichkeitsgrenze kommt. Hierbei ist es allerdings erforderlich, dem Kristallwachstum durch ständige Dispergierung entgegen zu wirken. Der hohe Gehalt an Calciumpropionat vermindert den Trocknungs-aufwand. Durch konvektive Trocknung der Kristallmaische wird das Zusammenbacken von Kristallaggregaten vermieden. Das erfindungsgemäße Verfahren ist somit energetisch günstig, vermeidet einen zusätzlichen Mahlschritt und eine aufwendige Feststoffhandhabung.

Eine feinteilige Suspenion bedeutet im Rahmen der Erfindung, daß das Dispergens als Feststoff mit einer mittleren Partikelgröße kleiner 100 µm, vorzugsweise von etwa 30 bis 60 µm vorliegt.

Ein feinteiliger Feststoff ist ein pulverförmiger Feststoff mit einer mittleren Partikelgröße kleiner 100 µm, vorzugsweise von etwa 30 bis 60 µm vorliegt.

Unter 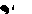 pumpbar" wird im Rahmen der Erfindung eine Lösung oder Suspension verstanden, deren Viskosität (beziehungsweise effektive Viskosität bei Suspensionen) noch niedrig genug ist, um mit Hilfe einer Pumpe gefördert werden zu können.

Ein erfindungsgemäßer konvektiver Trockner ist zum Beispiel ein Sprühturm, ein Sprühwirbelbett oder eine Kombination aus beiden, also entweder ein Sprühturm mit nachgeschaltetem Sprüh-Wirbelschichttrocker zur Nachagglomeration oder eine Sprühturm-Variante, in die ein Wirbelbett bereits integriert ist [FSD (Fluidized Bed Dryer, Ausführung der Fa. GEA Niro A/S, DK-Kopenhagen-Soeborg); SBD (Spray-Bed Dryer, Ausführung der Fa. APV Anhydro A/S, DK-Kopenhagen-Soeborg)]. In Abhängigkeit von der Trocknerausführung handelt es sich bei dem Endprodukt Calciumpropionat um ein feinteiliges Pulver, um ein Agglomerat oder um ein Granulat.

Agglomerate sind Feststoffpartikel, die durch Aneinanderlagerung noch feuchter feiteiliger Partikel (und anschließender Ausbildung von Feststoffbrücken) entstanden sind. Sie besitzen eine lockere sogenannte 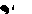 Brombeerstruktur". Ihre mittlere Partikelgröße liegt typischerweise zwischen 100 und 300 µm.

Granulate sind Feststoffpartikel, die durch Aufsprühen einer Lösung auf bereits gebildete Teilchen und anschließender Trockunung entstanden sind. Sie weisen eine kompaktere 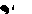 Zwiebelschalenstruktur" auf. Ihre mittlere Partikelgröße liegt typischerweise zwischen 300 und 500 µm.

In einer weiteren Ausführungsform des erfindungsgemäßen Verfahrens weist das Calciumpropionat-Pulver eine mittlere Partikelgröße von 30 bis 60 µm auf, wobei der Bereich von 40 bis 50 µm besonders bevorzugt ist. Die mittlere Partikelgröße ist ein auf den Massen- bzw. Volumenbeitrag der jeweiligen Partikel bezogener Wert (der Medianwert), der im Rahmen der Erfindung durch typische Partikelmeßverfahren (zum Beispiel Streulichtmessung, Laserlichtbeugung) bestimmt wird.

In einer weiteren Ausführungsform des erfindungsgemäßen Verfahrens weist die Calciumpropionat-Suspension einen Calciumpropionat-Anteil von 31 bis 60 % (w/w) oder von 35 bis 60 % (w/w), vorzugsweise von 40 bis 55% (w/w) oder von 43 bis 55 % (w/w), insbesondere von 45 bis 50% (w/w) auf.

In einer weiteren Ausführungsform des erfindungsgemäßen Verfahrens wird Calciumpropionat durch Umsetzung einer Calciumverbindung mit Propionsäure hergestellt.

In einer weiteren Ausführungsform des erfindungsgemäßen Verfahrens handelt es sich bei der Calciumverbindung um Calciumoxid, Calciumhydroxid oder Calciumcarbonat oder Gemischen daraus.

In einer weiteren Ausführungsform des erfindungsgemäßen Verfahrens erfolgt die Umsetzung zu Calciumpropionat diskontinuierlich, die Trocknung kontinuierlich.

Bevorzugt wird die Suspension im Kreislauf geführt.

In einer weiteren Ausführungsform des erfindungsgemäßen Verfahrens wird die Suspension unter Verwendung einer Dispergiermaschine hergestellt, die in den Kreislauf integriert ist und die Calciumverbindung in den Kreislauf einzieht. Ein Beispiel einer solchen Dispergiermaschine sind die Maschinen des Typs Conti-TDS der Fa. Ystral, Ballrechten-Dottingen.

Ein weiterer Gegenstand der Erfindung ist eine Vorrichtung zur Durchführung des erfindungsgemäßen Verfahrens, aufweisend
- einen Reaktor, bestehend aus einem Rührkessel oder einer Kaskade aus mindestens zwei Rührkesseln, eine
- Schlaufe und eine
- Dispergiermaschine zur Herstellung einer Suspension eines Feststoffs in einer Flüssigkeit,
- wobei der Auslaß der Dispergiermaschine über einen Schlaufenabschnitt mit einem Einlaß des Reaktors verbunden ist, ein Auslaß des Reaktors über einen weiteren Schlaufenabschnitt mit einem Einlaß der Dispergiermaschine verbunden ist, und
- eine Zuleitung mit einem weiteren Einlaß der Dispergiermaschine verbunden ist, die den Einzug eines Edukt-Feststoffes durch die Dispergiermaschine ermöglicht.

Eine weitere Ausführungsform der erfindungsgemäßen Vorrichtung weist zusätzlich auf
- einen Vorlagebehälter für die Aufnahme einer Produktsuspension, aufweisend einen Einlaß, der den Vorlagebehälter mit einem oben beschriebenen Auslaß für die Ableitung einer Produktsuspension verbindet, und einen Auslaß, der über eine weitere Zuleitung mit einem
- konvektiven Trockner verbunden ist.

Bevorzugt handelt es sich bei dem Trockner um einen Sprühtrockner, um ein Sprühwirbelbett oder um eine Kombination aus beiden Vorrichtungen.

In einer weiteren Ausführungsform der erfindungsgemäßen Vorrichtung weist der Trockner mindestens eine Einstoffdüse, eine Zweistoffdüse oder einen Rotationszerstäuber auf.

Die Erfindung wird näher durch die Zeichnung beschrieben.

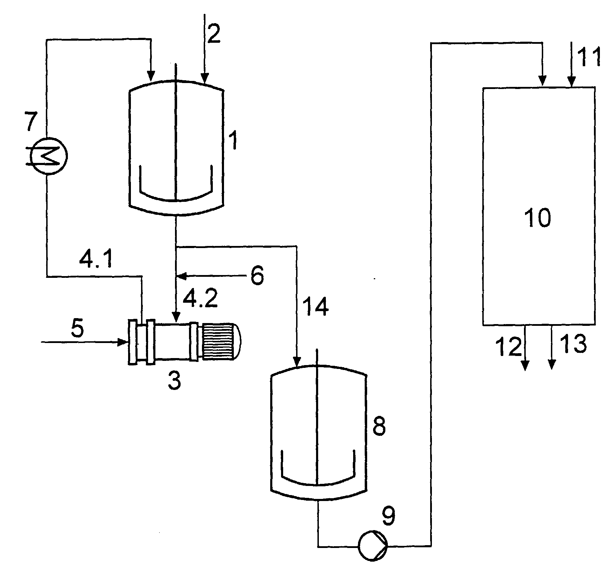

Es zeigt
- **Fig. 1**: eine Vorrichtung zur Durchführung des erfindungsgemäßen Verfahrens.

In Fig. 1 wird eine Vorrichtung zur Durchführung des erfindungsgemäßen Verfahrens dargestellt, bei der die Umsetzung einer Calciumverbindung mit Propionsäure diskontinuierlich, die Trocknung des Reaktionsproduktes kontinuierlich erfolgt. In einen Rührkessel **1** wird zunächst über eine Zuleitung **2** Wasser vorgelegt. Mit Hilfe einer Dispergiermaschine **3**, die zugleich als Pumpe wirkt, werden Wasser und später die wäßrige Reaktionsmischung in einer Schlaufe **4** gefördert. Dabei wird zunächst der feinteilige Kalk (Calciumhydroxid, Calciumoxid oder Calciumcarbonat) über eine Zuleitung **5** von der Dispergiermaschine eingezogen und in den Wasserkreislauf hineindispergiert, so daß eine feinteilige Kalkmaische entsteht. Anschließend oder auch zeitgleich wird die stöchiometrisch erforderliche Menge an Propionsäure über Zuleitung **6** in den Kreislauf gegeben und mit der Dispergiermaschine ebenfalls dispergiert. Im Kreislauf und im Rührkessel findet die Umsetzung zu Calciumpropionat statt. Durch die Dispergiermaschine wird sichergestellt, daß es auch bei der Überschreitung der Löslichkeitsgrenze nicht zur Bildung großer Kristalle kommt, sondern daß die auskristallisierten Calciumpropionat-Teilchen fein bleiben. Dadurch entsteht eine gleichmäßige Calciumpropionat-Suspension. Das Rühren im Rührkessel stellt sicher, daß die Suspension auch über einen längeren Zeitraum hinweg nicht sedimentiert. Die Reaktionswärme wird im Wärmeüberträger **7** abgeführt.

Wenn die beiden Reaktanden vollständig umgesetzt sind und der angestrebte pH-Wert eingestellt ist, ist die Reaktion abgeschlossen. Die Calciumpropionat-Suspension wir in einen weiteren Rührkessel **8**, der als Vorlagebehälter für die Trocknung dient, abgelassen. Von dort wird sie kontinuierlich mit einer Pumpe **9** zu einem konvektiven Trockner **10** gefördert. Als Trockenmedium dient ein erhitztes Gas (Luft oder inertisiertes Gas), welches über Zuleitung **11** zum Trockner geführt wird. Getrocknetes Calciumpropionat wird kontinuierlich über Ableitung **12** aus dem Trockner abgezogen. Das mit Wasserdampf beladene Trocknungsgas tritt über Ableitung **13** ebenfalls aus dem Trockner aus.

Beim konvektiven Trockner kann es sich um einen Sprühturm, ein Sprühwirbelbett oder eine Kombination aus Sprühturm und Wirbelbett handeln.
Wird ein Sprühturm verwendet, so wird das Calciumpropionat gemeinsam mit dem Trocknungsgas aus dem Trockner ausgetragen. In diesem Falle ist zusätzlich eine Abscheidevorrichtung vorzusehen, welche das Calciumpropionat vom Gas trennt, so daß Calciumpropionat und Trocknungsgas durch zwei getrennte Ableitungen **12** und **13** getrennt abgezogen werden können.

Die Erfindung wird näher durch die nachfolgenden Beispiele beschrieben.

### Beispiel 1:

In einem Rührbehälter wurden 2333kg Wasser vorgelegt. Mit Hilfe einer neben dem Rührbehälter aufgebauten Dispergiermaschine wurde das Wasser im Kreis gefördert. Gleichzeitig wurden nacheinander zwei Big Bags, die jeweils mit 500kg Calciumhydroxid gefüllt waren, an die Dispergiermaschine angeschlossen. Der Kalk wurde jeweils innerhalb von 300 Sekunden vollständig in den Wasserkreislauf eingezogen und von der Dispergiermaschine so gut dispergiert, daß bei einer anschließenden Siebung der entstandenen 43%-igen Kalksuspension mit einem Sieb mit 1 mm Maschenweite keine Grobpartikel nachgewiesen werden konnten.

### Beispiel 2:

In einem Rührbehälter (800 l) wurden 260 kg Wasser vorgelegt. Über eine Dispergiermaschine wurden 100 kg Calciumhydroxid eingezogen und in das in der Schlaufe befindliche Wasser dispergiert. Gleichzeitig wurden 200 kg Propionsäure mit Hilfe einer Schlauchpumpe in den Kreislauf gefördert. Nach einer Stunde war die Reaktion abgeschlossen. Die hierbei gebildete 45%-ige Calciumpropionat-Suspension wurde anschließend mit Hilfe einer Schlauch-pumpe zu einem Sprühtrockner (Innendurchmesser 1200 mm) gefördert, mit einem Rotationszerstäuber fein in einem Gasstrom (530 kg/h Stickstoff, 300 °C Eintrittstemperatur, 110 °C Austrittstemperatur) verteilt und zu einem feinteiligen Pulver (mittlere Partikelgröße 40 µm, Schüttdichte 300 kg/m³) getrocknet. Das Pulver wurde über einen Zyklon vom Trocknungsgas abgeschieden. Die Ausbeute betrug 220 kg Calciumpropionat.

### Beispiel 3:

In einer Technikumsapparatur desselben Aufbaus wurden 160 kg Wasser vorgelegt. Mit Hilfe der Dispergiermaschine wurden auch hier 100 kg Calciumhydroxid gemeinsam mit 200 kg zugeförderter Propionsäure in der Schlaufenflüssigkeit dispergiert. Die bei dieser Reaktion gebildete 55%-ige Calciumpropionat-Suspension war ebenfalls mit der Schlauchpumpe sehr gut pumpfähig. Sie wurde im Sprühtrockner bei 240°C Gaseintrittstemperatur zu einem feinteiligen Pulver getrocknet.

### Beispiel 4:

1 kg des Calciumpropionatpulvers des Beispiels 2 wurden in einem Laborwirbelbett, Durchmesser 140 mm, vorgelegt und mit 0,130 kg einer 30 %-igen wäßrigen Calciumpropionatlösung besprüht, während gleichzeitig mit 200kg/h Luft (200 °C Eintrittstemperatur) fluidisiert und getrocknet wurden. Danach wurde die mittlere Partikelgöße auf 200 µm vergrößert und zugleich die Schüttdichte auf 480 kg/m³ erhöht.

## Patentansprüche

1. Verfahren zur Herstellung von pulverförmigen Calciumpropionat unter Verwendung eines konvektiven Trockners, bei dem eine feinteilige, pumpbare Suspension von Calciumpropionat in Wasser mit einem Calciumpropionat-Gehalt von größer oder gleich 30 % (w/w) einem konvektiven Trockner zugeführt wird.

2. Verfahren nach Anspruch 1, dadurch gekennzeichnet, daß die Calciumpropionat-Suspension einen Calciumpropionat-Anteil von 31 bis 60 % (w/w) aufweist.

3. Verfahren nach Anspruch 1, dadurch gekennzeichnet, daß die Calciumpropionat-Suspension einen Calciumpropionat-Anteil von 40 bis 55 % (w/w) aufweist.

4. Verfahren nach Anspruch 1, dadurch gekennzeichnet, daß die Calciumpropionat-Suspension einen Calciumpropionat-Anteil von 45 bis 50 % (w/w) aufweist.

5. Verfahren nach Anspruch 1, dadurch gekennzeichnet, daß Calciumpropionat durch Umsetzung von Propionsäure mit einer Calciumverbindung, ausgewählt aus Calciumoxid, Calciumhydroxid, Calciumcarbonat oder Gemischen daraus, hergestellt wird.

6. Verfahren nach Anspruch 5, dadurch gekennzeichnet, daß die Umsetzung zu Calciumpropionat diskontinuierlich, die Trocknung der Suspension kontinuierlich erfolgt.

7. Verfahren nach Anspruch 1, dadurch gekennzeichnet, daß die Suspension in einem Kreislauf geführt wird.

8. Verfahren nach Anspruch 7, dadurch gekennzeichnet, daß die Suspension unter Verwendung einer Dispergiermaschine hergestellt wird, die in den Kreislauf integriert ist und die Calciumverbindung in den Kreislauf einzieht.

9. Vorrichtung zur Durchführung des Verfahrens nach einem der Ansprüche 1 bis 8, gekennzeichnet durch
- einen Reaktor (1), bestehend aus einem Rührkessel oder einer Kaskade aus mindestens zwei Rührkesseln, durch
- eine Schlaufe (4) und
- eine Dispergiermaschine (3) zur Herstellung einer Suspension eines Feststoffs in einer Flüssigkeit, wobei der Auslaß der Dispergiermaschine (3) über einen Schlaufenabschnitt (4.1) mit einem Einlaß des Reaktors (1) verbunden ist, ein Auslaß des Reaktors (1) über einen weiteren Schlaufenabschnitt (4.2) mit einem Einlaß der Dispergiermaschine verbunden ist, und eine Zuleitung (5) mit einem weiteren Einlaß der Dispergiermaschine (3) verbunden ist, die den Einzug eines Edukt-Feststoffes durch die Dispergiermaschine (3) ermöglicht.

10. Vorrichtung nach Anspruch 9, dadurch gekennzeichnet, daß die Schlaufe (4) einen Einlaß für die Zuleitung (6) einer Eduktflüssigkeit und einen Auslaß für die Ableitung (14) einer Produktsuspension aufweist.

11. Vorrichtung nach Anspruch 10, zusätzlich aufweisend
- Vorlagebehälter (8) für die Aufnahme einer Produkt-Suspension, aufweisend einen Einlaß, der Vorlagebehälter (8) mit einem Auslaß (14) für die Ableitung einer Produktsuspension gemäß Anspruch 10 verbindet, und einen Auslaß, der über eine weitere Zuleitung mit einem
- konvektiven Trockner (10) verbunden ist.

12. Vorrichtung nach Anspruch 11, dadurch gekennzeichnet, daß es sich bei dem Trockner (10) um einen Sprühtrockner, um ein Sprühwirbelbett oder um eine Kombination aus beiden Vorrichtungen handelt.

13. Vorrichtung nach Anspruch 11, dadurch gekennzeichnet, daß der Trockner (10) mindestens eine Einstoffdüse, Zweistoffdüse oder ein Rotationszerstäuber aufweist.
